# EUROPÄISCHE PATENTSCHRIFT

(11) **EP 0 396 520 B1**
(45) Veröffentlichungstag und Bekanntmachung des Hinweises auf die Patenterteilung: **02.06.1993**
(21) Anmeldenummer: 90850147.1
(22) Anmeldetag: 20.04.1990
(51) Int. Cl.: A61F 2/42, A61F 2/36

(54) **Verankerungselement zum Halten eines Gelenkteils eines Fussgelenks oder anderer künstlicher Gelenke**
Anchor element for holding a joint component of an ankle joint or other artificial joints
Elément d'ancrage pour supporter une partie articulaire d'une articulation tibiotarsienne ou d'autres articulations artificielles

(30) Priorität: 25.04.1989 SE 8901509; 13.09.1989 US 406587
(43) Veröffentlichungstag der Anmeldung: 07.11.1990
(60) Teilanmeldung: 92201843.7
(73) Patentinhaber: Branemark, Per-Ingvar, S-431 39 Mölndal (SE)
(72) Erfinder: Branemark, Per-Ingvar, S-431 39 Mölndal (SE)
(74) Vertreter: Barnieske, Hans Wolfgang

(56) Entgegenhaltungen:
- EP-A- 0 099 167
- WO-A-79/00739
- DE-A- 2 808 740
- DE-B- 2 338 136
- DE-U- 8 812 806
- FR-A- 2 595 565
- US-A- 4 488 319

## Beschreibung

### Hintergrund und Gebiet der Erfindung

Die vorliegende Erfindung betrifft ein Verankerungselement zum Halten eines Gelenkteils eines künstlichen Gelenks und ein künstliches Gelenk.

Die bevorzugten Ausführungsarten werden im folgenden bezugnehmend auf die Wiederherstellung von Fußgelenken beschrieben. Die Erfindung ist jedoch darauf nicht begrenzt. Die Erfindung kann auch bei ähnlichen Gelenken, besonders bei lasttragenden Gelenken, eingesetzt werden. Die Erfindung kann auch bei Wiederherstellungen nach Amputation oder anderen Verletzung verwendet werden.

### Beschreibung verwandter Gebiete

Über eine beträchtliche Zeitspanne wurden Versuche unternommen, zerstörte Gelenke durch künstliche Prothesen zu ersetzen. Diese sind in dem Knochengewebe entweder direkt oder mittels einer zwischenliegenden, adhäsiven Schicht, üblicherweise Knochenzement, verankert werden. Für die Prothesen wurden verschiedene Materialien, wie rostfreier Stahl, Chrom- und Kobaldlegierungen, Titanlegierungen, Aluminiumlegierungen, keramische Werkstoffe, Kohlefaser und viele andere Materialien verwendet. Man hat eine große Erfindungsgabe auf die Oberfläche und die Oberflächenbeschaffenheiten aufgewendet, von denen man annahm, daß man die bestmögliche Stabilität der Verankerung erreicht.

Das größte Problem der orthopädischen Chirurgie mit Prothesen ist nach wie vor, wie gut die Komponenten der Prothese im Knochengewebe des Patienten gesichert sind. Experimente, die eine feste mechanische Verbindung zwischen Implantat und Knochengewebe mit sich bringen ("Preß-Fit", vgl. EP 149 527), und die Verwendung von Knochenzement (Polymethylmethacrylat) haben nicht zu zufriedenstellenden Ergebnissen geführt.

Konventionellerweise werden die Prothesen im Knochenzement gesichert. Es wurde große Sorgfalt darauf verwendet, das Knochenmark von dem freigelegten Knochen zu entfernen - der Hohlraum ist sogar sauber ausgespült worden, bevor die Prothese eingesetzt und dann in dem Knochenzement gesichert wurde. Die einzementierten Prothesen lockern sich jedoch oft. Dies ist besonders bei Prothesen der Fall, die in ein lasttragendes Gelenk, wie Fußgelenke und Hüftgelenke, einzementiert sind. Es ist auch herausgefunden worden, daß Knochenzement, wenn er vor dem Hartwerden eingesetzt wird, dazu neigt in das angrenzende Knochengewebe einzusickern. Davon ausgehend, bietet dieses Einsickern eine gewisse mechanische Stabilität, aber bei jungen, übergewichtigen oder aktiveren Personen, wird das Gelenk zwischen Knochen und Zement letztendlich aufgrund von Mikro-Verschiebungen zerstört. Es wächst eine Schicht eines Verbindungsgewebes zwischen Prothese und Knochen und schließlich lockert sich das Gelenk.

Ein anderes heute angewandtes Verfahren ist eine zementlose Methode, die auf eine biologische Sicherung der Prothese abzielt, z. B. wird ein direkter Kontakt zwischen Prothese und Knochengewebe ohne Zwischenschicht eines Verbindungsgewebes oder einer adhäsivwirkenden Schicht gewünscht. Um eine zufriedenstellende Stabilität der Verankerung zu erreichen, sind verschiedene Materialien und Oberflächenstrukturen für die Prothese ausgetestet worden und es wurde eine Technik angewendet, die als "Preß-Fit" bekannt ist, um die Prothese nach dem Einsetzen in ihrer Position festzuhalten. Kürzlich veröffentliche Experimente (vgl. Schimmel et Huiskes, "Primary fit of the Lord cementless total hip", Acta. Orthop. Scand. 1988: 59(6): 638-642 lassen jedoch erkennen, daß diese Methode in Wirklichkeit nicht funktioniert. Es hat sich gezeigt, daß diese Methode zu einer extrem ungünstigen Drei-Punkte-Belastung mit Spannungskonzentrationen führt (EP 176 711).

Das Problem kann nicht bloß dadurch gelöst werden, daß eine "geeignete Oberfläche eines Materials" ausgewählt wird. Es gibt eine Reihe anderer Faktoren, die von entscheidender Bedeutung sind.

Dazu werden in den vergangenen Jahren Versuche unternommen, Halterungen aus Titan in die Knochenmarkshöhlung des Knochens zu verankern, um verknöchernd einzuwachsen, wie es von Hagert et al. "Metalcarpophalangal Joint Replacement with Osseo-integrated Endoprostheses" in Scand. J. Plast. Reconstr. Surg 20: Seiten 207-218, 1986, beschrieben ist.

Es ist bekannt orale und extra-orale Prothesen im Knochengewebe darauf zu verankern. Diese Technik des verknöchernden Zusammenwachsens in der Zahnmedizin wurde von Prof. Brånemark und seinen Kollegen in den letzten 25 Jahren mit ausgezeichneten Ergebnissen entwickelt, in-dem Halterungen in den Backenknochen zum Halten von Zähnen und Brücken eingesetzt werden. Die Experimente jedoch, die von Hagert durchgeführt wurden um diese Technik auf die Wiederherstellung von Fingergelenken zu übertragen, haben die Erwartungen nicht erfüllt. Die unakzeptabelen Ergebnisse sind offenbar auf die vollkommen unterschiedlichen Bedingungen zurückzuführen, die bei der Verwendung der "Technik der Zahnmedizin" bei der prothetischen Wiederherstellung von Fingergelenken auftreten. Diese Probleme sind selbstverständlich im Fall von Fußgelenk- und Hüftgelenk-Prothesen noch erschwert, nachdem vollkommen verschiedene Lasten auf diese Gelenke wirken.

Heute besteht das hauptsächliche Problem in der orthopädischen Chirurgie mit Prothesen nach wie vor darin, daß sich die Knochenverankerungseinheit lockert. Jedoch treten mit einer Erfolgsrate von mehr als 90 % über einen Zeitraum von 20 Jahren eine Reihe anderer Probleme auf, die insoweit, bisher nicht in Betracht gezogen werden mußten. Eines der hauptsächlichen Probleme ist eine zunehmende Abnutzung des Gelenkteils. Es ist eine andere Art der Ausführung von Prothesen im Vergleich zur bisher gebräuchlichen gefragt, wenn das verknöchernd zusammenwachsende Verfahren angewendet werden soll. Um das Gelenkteil austauschen zu können, ohne die Knochenverankerung zu beschädigen, muß das System der Prothese in Komponenten aufgeteilt werden, bei denen das Gelenkteil von dem tatsächlichen Verankerungselement abgetrennt werden kann. Darüber hinaus, wenn das zweistufige Verfahren angewendet wird, muß es möglich sein, das Gelenkteil in einem zweiten Schritt zu verbinden, wenn der Patient, oder zumindest das künstliche Gelenk des Patienten, nicht unbeweglich werden soll. Dazu mussen zwei Umstände in Betracht gezogen werden: Erstens, das Gelenkteil unterliegt der Abnutzung und muß deshalb austauschbar sein. Zweitens muß das Gelenkteil austauschbar sein, um das zweistufige Verfahren anwenden zu können.

Aus FR-A-2 595 565 ist ein Verankerungselement zum Halten eines Gelenkteils eines künstlichen Hüftgelenks bekannt, wobei das Verankerungselement eine hohle Führungs- und Zentrierbuchse zum Halten des Gelenkteils aufweist und aus einem korrosionsbeständigen Material, vorzugsweise Titan, geformt ist, um eine dauerhafte Verankerung zu erreichen, mit einer Frontplatte, aus der die Führungs- und Zentrierbuchse herausragt, wobei die Frontplatte ein oder mehrere Löcher zur Aufnahme von Befestigungsschrauben aufweist.

Aus DE-A-28 08 740 ist eine dauerhaft im Körper verankerbare Gelenkprotese bekannt, deren in die Knochensubstanz reichenden Teile von einem knochenfreundlichen Implantatmaterial umhüllt sind.

### Zusammenfassung der Erfindung

Es ist nun überraschenderweise festgestellt worden, daß die Probleme und Nachteile der oben beschriebenen Techniken durch die vorliegende Erfindung eliminiert werden können.

Die Erfindung betrifft ein Verankerungselement zum Halten eines Gelenkteils eines künstlichen Gelenks, wobei das Verankerungselement eine zumindest teilweise hohle Führungs- und Zentrierbuchse zum Halten des Gelenkteils aufweist, wobei das Verankerungselement aus einem Material geformt ist, das sich mit dem Knochen- und Knochenmarkgewebe verträgt, wobei das Verankerungselement eine Oberfläche hat, die zumindest teilweise verknöchernd mit dem Gewebe zusammenwachsen kann, um eine dauerhafte Verankerung dem Knochen- und Knochenmarkgewebe zu erreichen.

Die Erfindung betrifft auch ein künstliches Gelenk.

Andere Eigenschaften und Vorteile der Erfindung werden mit der folgenden Beschreibung der bevorzugten Ausführungen der Erfindung mit dem Hinweis auf die begleitenden Zeichnungen deutlich.

### Kurze Beschreibung der Zeichnungen

- Fig. 1: ist eine Explosionszeichnung eines Verankerungselementes nach einer ersten Ausführungsform der Erfindung;
- Fig. 2: ist eine Explosionszeichnung eines Verankerungselementes nach einer zweiten Ausführungsform der Erfindung. Das Verankerungselement der Fig. 2 entspricht generell dem Verankerungselement nach Fig. 1, außer, dass das Verankerungselement nach Fig. 2 keine flügelartige Platte hat und in einer umgekehrten Lage angeordnet ist;
- Fig. 3: ist eine perspektivische Ansicht einer ersten Stufe bei der Wiederherstellung eines Fußgelenkes unter Verwendung der Verankerungselemente der Fig. 1 und 2;
- Fig. 4: ist eine perspektivische Ansicht, ähnlich wie Fig. 3, nach einer fertigen Wiederherstellung.

### Beschreibung der bevorzugten Ausführungen

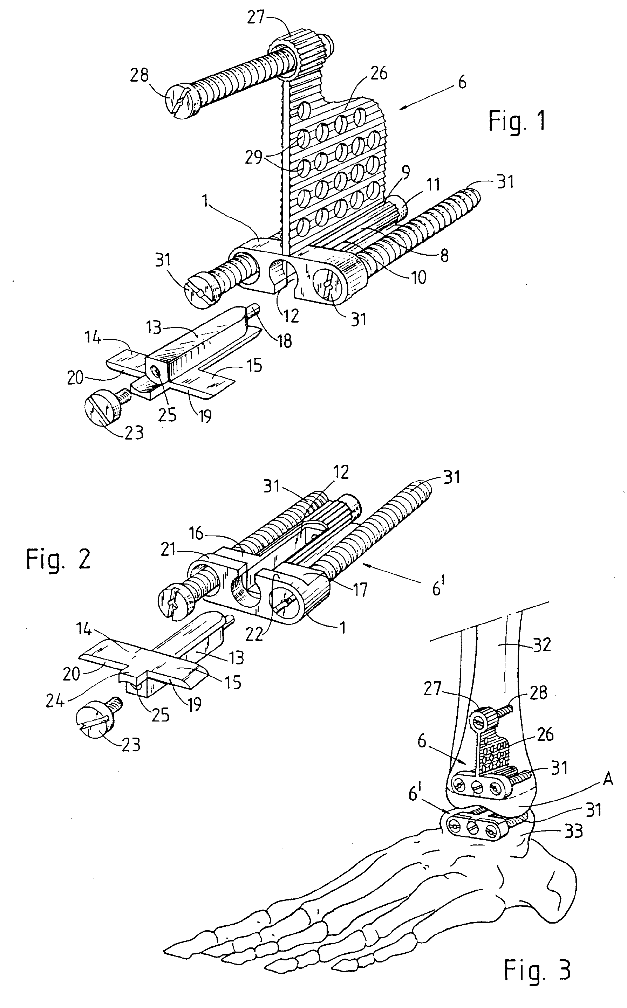

Bezugnehmend auf Fig. 1 weist ein nicht-rotationssymmetrisches Verankerungselement 6 nach einer ersten Ausführungsart der Erfindung eine Frontplatte 1 auf. Von der Frontplatte 1 ragt rückseitig eine hohle, mittig sitzende Führungs- und Aufnahmebuchse 8 heraus. Die Buchse 8 ist im wesentlichen zylinderförmig und ist mit einer längsverlaufenden Riffelung an ihrer radialen äußeren Oberfläche versehen. Die Riffelung fördert das Zusammenwachsen. Die Riffelung kann zweckmäßigerweise identisch sein zu der eines axialen Ansatzes konisch-verjüngter, rotationssymmetrischer Halterungen, die in der Zahnmedizin verwendet werden. Das Ende 9 der Buchse 8, das der Frontplatte 1 abgekehrt ist, hat ein Innengewinde, das zu einem Außengewinde eines Bolzens 11 paßt. Die dem Gelenk zugewandte Seite der Buchse 8 ist geschlitzt, wobei ein Hohlraum 12 für die Aufnahme eines Einsatzstückes 13 in einem ersten Schritt (wie unten beschrieben) freigelegt wird. Die Rückseite des Einsatzstückes 13 hat einen Zapfen 18, der in eine entsprechende Ausnehmung (nicht gezeigt) in den Bolzen 11 paßt, wenn das Einsatzstück an Ort und Stelle sitzt. Von der Frontseite des Einsatzstückes ragen im wesentlichen rechtwinkelig Schenkel 14, 15 nach außen. Wenn das Einsatzstück 13 an Ort und Stelle sitzt, passen die Schenkel 14, 15 mit zurückgenommenen flachen Oberflächen 16, 17 (Fig. 2) auf jeder Seite des Schlitzes und des Hohlraumes 12 zusammen. Die Frontkanten 19, 20 der Schenkel 14, 15 werden folglich mit Vorsprüngen 21, 22 an der Frontplatte 1 in Verbindung gebracht, wenn das Einsatzstück 13 letztendlich mit einer Schraube 23, die mit einer Bohrung 25 auf der Stirnseite des Einsatzstückes 13 zusammenwirkt, positioniert wird. Das Einsatzstück 13 hat auch einen frontseitigen Ansatz 24. Die Frontplatte 1 hat zwei Löcher, auf jeder Seite der Buchse 8 eines, um die Befestigungsschrauben 31 aufzunehmen.

Das Verankerungselement 6 (Fig. 1) weist eine mittige, flügelähnliche Platte 26 auf, die aus der Buchse 8 herausragt. Das Verankerungselement 6' (Fig. 2) hat eine solche Platte nicht. Das obere Ende der Platte 26, das der Buchse 8 abgekehrt ist, hat eine Führungsbuchse 27 für die Aufnahme einer Schraube 28. Beides, die Platte 26 und die Führungsbuchse 27, sind mit einer längsverlaufenden Riffelung versehen, welche, wie die Riffelung 10, sich in der Einführrichtung des Verankerungselementes 6 erstreckt. Die Riffelungen fördern das Zusammenwachsen. Die Platte 26 weist eine Mehrzahl von Löchern 29 auf. Die Löcher 29 ergeben Schneidkanten.

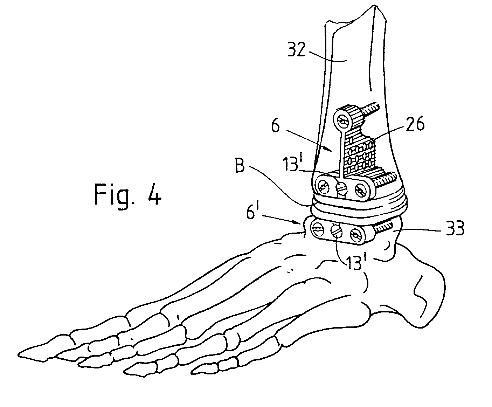

Insbesondere die Verankerungselemente 6, 6' der Fig. 1 und 2 sind vorzugsweise gänzlich aus Titan. Die Oberflächen des Einsatzstückes 13 und der Frontplatte 1 sind nicht geriffelt um ein Zusammenwachsen nicht zu fördern. Wenn das Verankerungselement 6 (oder 6') in das Knochengewebe einwächst, wird das Einsatzstück 13 entfernt und durch ein dauerhaftes Einsatzstück 13' (Fig. 4) mit einem künstlichen Gelenkteil B ersetzt.

Folglich, entsprechend Fig. 3, ermöglicht die Erfindung, daß das Originalgelenk A intakt bleibt, bis das Verankerungselement 6, 6' eingewachsen ist. Das defekte Gelenk A wird dann durch das künstliche Gelenk B ersetzt. Das künstliche Gelenk B ist zwischen den Verankerungselementen 6, 6', wie es in Fig. 4 dargestellt ist, positioniert.

Bei einer Operation wird die Haut über dem Schienbein 32 (oder dem Schienbeinknochen) und dem Sprungbein 33 (oder dem Fußknöchel des Sprungbeins) nahe am Gelenk A geöffnet und es werden zweckmäßige Mittelpunkte für den mittigen Ansatz des Verankerungselementes 6 bestimmt. Dann werden sorgfältig vier Löcher in das Schienbein 32 gebohrt und es werden sorgfältig drei entsprechende Löcher in das Sprungbein 33 gebohrt. Die Löcher in dem Schienbein 32 und dem Sprungbein 33 werden mit Schlitzen untereinander verbunden. Dann wird eine genaue Vorbereitung durchgeführt, wobei für die Verankerungselemente 6, 6' die letzte Vorbereitung darin besteht, wenn Schrauben 31, 28 eingeschraubt werden. Die Absätze in der Frontplatte 1 und in der Buchse 27 dienen als Führung für die Schrauben 31, 28. Die Schrauben 31, 28 ergeben zusätzliche Stabilität und positionieren die Verankerungselemente 6, 6'. Sobald die Verankerungselemente 6, 6' positioniert worden sind, wird ein glattpoliertes Einsatzstück 13 in die Buchse eines jeden Verankerungselementes 6, 6' angesetzt. Die Verankerungselemente 6, 6' werden dann in dem Knochen 32, 33 belassen, bis ein verknöcherndes Einwachsen stattfindet. Für Verankerungselemente, die in dem Knochen nahe eines Gelenkes zur Wiederherstellung eines Fußknöchels eines Sprungbeins eingesetzt sind, beträgt der Zeitraum zum Einwachsen zwischen drei und sechs Monate. Das Gelenk A ist während des Einwachsens der zwei Verankerungselemente 6, 6' vollkommen intakt.

Nach der Zeitdauer zum Einwachsen wird das verletzte oder abgenutzte Gelenk A entfernt und durch das künstliche Gelenkteil B ersetzt. Das künstliche Gelenkteil B wird zweckmäßigerweise mit Hilfe der Einsatzstücke 13' gesichert, die sich in den betreffenden Verankerungselementen 6, 6' gegenüberliegen. Alternativ dazu kann das künstliche Gelenk B im voraus mit zwei Einsatzstücken 13' vorbereitet werden. Im letzteren Falle werden die, während der Zeitdauer zum Einwachsen in der Führungsbuchse 8, gegenwärtigen Einsatzstücke 13 durch die neuen Einsatzstücke 13' ersetzt.

An Stelle der oben beschriebenen zweistufigen Vorgehensweise, kann das Fußgelenk selbstverständlich auch in einem Schritt wiederhergestellt werden. Bei einer solchen Wiederherstellung gibt es jedoch das Problem, das Fußgelenk zu belasten, bevor die Verankerungselemente 6, 6' genügend eingewachsen sind.

Die Verankerungselemente 6, 6' können auch im Vertrauen auf das automatische Einwachsen der Verankerungselemente 6, 6' in das Knochengewebe ohne die speziellen Verankerungsschrauben 31, 28 verwendet werden. In diesem Fall jedoch, wo Gelenke, wie Fußgelenke, eine bestimmte Belastung aufnehmen müssen, helfen diese Schrauben 31, 28 die Belastung zu verteilen und die Verankerungselemente 6, 6' genau zu positionieren.

Bei der Wiederherstellung eines Fußgelenkes, wie es in den Fig. 1 bis 4 dargestellt ist, liegen die Verankerungselemente 6, 6' quer zur Längsachse des Fußgelenkes. Die zwei Verankerungselemente 6, 6' benutzen das gleiche Grundprinzip, aber das Verankerungselement 6 für das Schienbein 32 hat den speziellen, stabilisierenden und verankernd wirkenden Flügel bzw. die Platte 26. Es gibt für eine solche Platte im Fußknöchel des Sprungbeins keinen Platz. Sogar in dem Schienbein 32 ist die Halteplatte 26 andererseits nicht dringend notwendig. Es könnten zwei Verankerungselemente 6' (derart, wie es in Fig. 2 dargestellt ist) verwendet werden, um die notwendige Wiederherstellung des Gelenkes zu erreichen.

Die Erfindung, mit dem stabilen, verknöchernden Einwachsen der Verankerungselemente 6, 6' in das Knochengewebe, basiert auf einer umfassenden, experimentellen, biologischen Analyse des Aufbaus und der Funktion der Gelenke im Verlauf einer Krankheit oder während einer Abwehrreaktion nach einer Zersetzung des Knochengewebes durch Abnutzung oder Entzündung und umfassenden Studien der Blutzufuhr zum Knochenmark. Es ist nachgewiesen worden, daß der synthetische Ersatz zerstörter, künstlicher Knorpel und Sehnen auf dem Grundprinzip aufbauen muß, das Knochen- und Knochenmarksgewebe eine Einheit in Bezug auf Aufbau und Funktionsweise bilden. Ein Zusammenwirken der beiden Komponenten, wenigstens über einen längeren Zeitraum betrachtet, ist unbedingt nötig, wenn das harte Gewebe als tragendes Element wirken soll.

Die Erfindung basiert folglich auf der Verwirklichung, daß bei der Verankerung eines prothetischen Austausches einer Gelenkoberfläche und von Sehnen zu Teilen des Knochengerüstes in Gelenknähe, die Zusammenwirkung zwischen Knochenmark und Knochengewebe berücksichtigt werden muß. Befestigungselemente, die zu diesem Zweck entsprechend der Erfindung verwendet werden, müssen folglich aus einem Material bestehen, das sich mit dem Gewebe verträgt und müssen mit einer Oberflächenstruktur versehen sein, die ein fehlerfreies, biologisches Zusammenwachsen mit dem Knochen- und Knochenmarksgewebe sicherstellt. Natürlich fördern Löcher 29 in der Platte 26 ein solches Einwachsen besonders. Wenn das Verankerungselement eingesetzt wird, führen die Kanten der Löcher 29 eine Schneidfunktion aus und gewährleisten, daß sich die Basisplatte gut in das umgebende Gewebe einfügt und das ein Teil des Gewebes durch die Löcher 29 hindurch gerichtet ist, womit sich ein entsprechendes Einwachsen des Verankerungselementes 6 in seine Umgebung ergibt.

Die Verankerungselemente wirken als Vorbereitungsinstrument, wenn sie in dem Gewebe eingesetzt sind. Alternativ dazu können spezielle Instrumente zur Vorbereitung benutzt werden, um optimale Bedingungen zum Enwachsen in den Knochen zu erreichen und können als zusätzliche Halterung und weiterhin zur Optimierung der Stabilität dort belassen werden. Das Einwachsen hängt teilweise von den Schneidkanten der Verankerungselemente ab, die eine perfekte Übereinstimmung mit dem umgebenden Gewebe ergeben.

Das Grundprinzip der vorliegenden Erfindung ist folglich die Erkenntnis, daß Knochen- und Knochenmarksgewebe eine strukturelle und funktionelle Einheit bilden, und die Wechselwirkung zwischen ihnen diesbezüglich berücksichtigt werden muß, wenn ein chirurgischer Austausch vorgenommen werden soll. Das bedeutet, daß soweit wie technisch möglich, ein Verbindungsweg zwischen Knochenmark und Knochengewebe ermöglicht werden muss.

Die oben erwähnten Verankerungsschrauben können wie die Verankerungselemente, die in meiner anhängigen US-Anmeldung US 406 486 gezeigt wird, geformt sein, welche als ein hohler, im wesentlichen zylinderbuchsenartiger Körper mit einem Außengewinde und, falls zweckmässig, auch mit abnehmender Wanddicke, ausgebildet ist. Das offene Ende des Körpers kann konisch verjüngt sein und kann mit Schlitzen, die am offenen Ende anfangen, versehen sein. Es wird somit ein selbständiges Gewindeschneiden erreicht, wenn der schraubenartige Körper in den Knochen eingeschraubt wird und das entfernte Knochengewebe innerhalb der Schraube aufgenommen wird um ein geeignetes, verknöcherndes Einwachsen sicherzustellen.

Obwohl die Erfindung in Verbindung mit einer bevorzugten Ausführungsart beschrieben worden ist, werden dem Fachmann viele andere Abwandlungen und Modifikationen und andere Einsatzmöglichkeiten eröffnet. Es wird deshalb vorgebracht, daß die Erfindung nicht durch diese spezielle Darlegung beschränkt ist, sondern nur durch die anhängenden Ansprüche.

## Patentansprüche

1. Verankerungselement (6; 6') zum Halten eines Gelenkteils eines künstlichen Gelenks, insbesondere eines Fussgelenks, wobei das Verankerungselement (6, 6') eine zumindest teilweise hohle Führungs- und Zentrierbuchse (8) zum Halten des Gelenkteils aufweist und vorzugsweise aus Titan geformt ist, mit einer Frontplatte (1), aus der die Führungs- und Zentrierbuchse (8) herausragt, wobei die Frontplatte (1) ein oder mehrere Löcher zur Aufnahme von Befestigungsschrauben (31) aufweist, **gekennzeichnet** durch einen Hohlraum (12) und einen in Buchsenlängsrichtung verlaufenden und den Hohlraum nach außen öffnenden, seitlichen Schlitz der Führungs- und Zentrierbuchse (8) zur austauschbaren Aufnahme eines Einsatzstückes (13') für die Verbindung des Gelenkteils mit dem Verankerungselement (6, 6'), das aus einem Material geformt ist, das sich mit Knochen- und Knochenmarkgewebe verträgt und eine Oberfläche hat, die zumindest teilweise verknöchernd mit dem Gewebe zusammenwachsen kann, um eine dauerhafte Verankerung in dem Knochen- und Knochenmarkgewebe zu erreichen, wobei der Hohlraum (12) und der seitliche Schlitz der Führungs- und Zentrierbuchse (8) sich durch die Frontplatte (1) hindurch fortsetzt.

2. Verankerungselement nach Anspruch 1, dadurch **gekennzeichnet**, dass das Verankerungselement (6, 6') eine im wesentlichen flache, flügelartige Platte (26) aufweist, die aus der Führungs- und Zentrierbuchse (8) herausragt.

3. Verankerungselement (6, 6') nach Anspruch 2, dadurch **gekennzeichnet**, dass die flache, flügelartige Platte (26) eine längsverlaufende Riffelung und Löcher (29) mit Schneidkanten hat.

4. Verankerungselement (6, 6') nach Anspruch 3, dadurch **gekennzeichnet,** dass eine zusätzliche Verankerungsschraube (28) in einem zur Aufnahme dieser Schraube (28) vorgesehenen Loch in einer Halterung (27) an einem der Führungs- und Zentrierbuchse (8) abgewandten Ende der flachen, flügelartigen Platte (26) angeordnet ist.

5. Verankerungselement (6, 6') nach einem der vorhergehenden Ansprüche, dadurch **gekennzeichnet,** dass die Befestigungsschrauben (31) ein Aussengewinde über die im wesentlichen gleiche Länge wie die Führungs- und Zentrierbuchse (8) haben.

6. Verankerungselement (6, 6') nach einem der vorhergehenden Ansprüche, dadurch **gekennzeichnet,** dass die Führungs- und Zentrierbuchse (8) eine längsverlaufende Riffelung hat, um das Einwachsen zu fördern.

7. Künstliches Gelenk, mit einem Gelenkteil, das von einer wenigstens teilweise hohlen Führungs- und Zentrierbuchse (8) eines Verankerungselements (6, 6') nach einem der Ansprüche 1 bis 6 gehalten wird.

## Claims

1. An anchoring element (6; 6') for supporting a joint mechanism of an artificial joint, more particularly an ankle joint, said anchoring element (6, 6') comprising an at least partially hollow guide and centring sleeve (8) for supporting the joint mechanism, said anchoring element being formed preferably from titanium, said anchoring element having a front plate (1) from which the guide and centring sleeve (8) protrudes, the front plate (1) having one or more holes to receive attachment screws (31), characterised by a hollow cavity (12) and a lateral slit in the guide and centring sleeve (8), said slit extending in the longitudinal direction of the sleeve and opening the hollow cavity to the exterior, for the replaceable reception of an insert (13') for the connection of the joint mechanism to the anchoring element (6, 6'), said insert being formed from a material compatible with the bone and marrow tissue and having a surface which can at least partially be osseo-integrated with the tissue to achieve permanent anchorage in the bone and marrow tissue, the hollow cavity (12) and the lateral slit in the guide and centring sleeve (8) continuing through the front plate (1).

2. An anchoring element according to claim 1, characterised in that the anchoring element (6, 6') has a substantially flat wing-like plate (26) which protrudes from the guide and centring sleeve (8).

3. An anchoring element (6, 6') according to claim 2, characterised in that the flat wing-like plate (26) has a longitudinally extending corrugation and holes (29) with cutting edges.

4. An anchoring element (6, 6') according to claim 3, characterised in that an additional anchoring screw (28) is disposed in a hole provided to receive said screw (28) in a fixture (27) at an end of the flat wing-like plate (26) facing away from the guide and centring sleeve (8).

5. An anchoring element (6, 6') according to any one of the preceding claims, characterised in that the attachment screws (31) have an external screwthread over substantially the same length as the guide and centring sleeve (8).

6. An anchoring element (6, 6') according to any one of the preceding claims, characterised in that the guide and centring sleeve (8) has a longitudinal corrugation to promote integration.

7. An artificial joint comprising a joint mechanism supported by at least partially hollow guide and centring sleeve (8) of an anchoring element (6, 6') according to any one of claims 1 to 6.

## Revendications

1. Elément d'ancrage (6; 6') pour le maintien d'une pièce d'articulation d'une articulation artificielle, notamment une articulation du pied, l'élément d'ancrage (6, 6') comportant une douille de guidage et de centrage (8) au moins partiellement creuse pour le maintien de la pièce d'articulation, et étant de préférence réalisé en titane, l'élément d'ancrage comportant également une plaque frontale (1) de laquelle fait saillie la douille de guidage et de centrage (8), la plaque frontale (1) comportant un ou plusieurs trous destinés à recevoir des vis de fixation (31), caractérisé par une cavité (12) et une fente latérale de la douille de guidage et de centrage (8), cette fente s'étendant dans la direction longitudinale de la douille et ouvrant la cavité vers l'extérieur, en vue de loger de manière interchangeable une pièce d'insert (13') pour la liaison de la pièce d'articulation à l'élément d'ancrage (6, 6') qui est réalisé en un matériau compatible avec le tissu osseux et le tissu médullaire, et présente une surface pouvant se souder aux tissus, au moins partiellement en s'ossifiant, afin d'obtenir un ancrage durable dans le tissu osseux et le tissu médullaire, la cavité (12) et la fente latérale de la douille de guidage et de centrage (8) se poursuivant au travers de la plaque frontale (1).

2. Elément d'ancrage selon la revendication 1, caractérisé en ce que l'élément d'ancrage (6, 6') comporte une plaque (26) essentiellement plane et en forme d'aile, qui fait saillie de la douille de guidage et de centrage (8).

3. Elément d'ancrage ( 6, 6') selon la revendication 2, caractérisé en ce que la plaque (26) plane et en forme d'aile, comporte un rainurage longitudinal et des trous (29) à arêtes vives.

4. Elément d'ancrage (6, 6') selon la revendication 3, caractérisé en ce qu'une vis d'ancrage supplémentaire (28) est disposée dans un trou destiné à recevoir cette vis (28) et prévu dans un support (27) à l'extrémité éloignée de la douille de guidage et de centrage (8), de la plaque (26) plane et en forme d'aile.

5. Elément d'ancrage (6, 6') selon l'une des revendications précédentes, caractérisé en ce que les vis de fixation (31) comportent un filetage extérieur sur une longueur sensiblement identique à celle de la douille de guidage et de centrage (8).

6. Elément d'ancrage (6, 6') selon l'une des revendications précédentes, caractérisé en ce que la douille de guidage et de centrage (8) comporte un rainurage longitudinal en vue de promouvoir le soudage aux tissus.

7. Articulation artificielle, comprenant une pièce d'articulation qui est maintenue par une douille de guidage et de centrage (8), au moins partiellement creuse, d'un élément d'ancrage (6, 6') selon l'une des revendications 1 à 6.
